# EUROPEAN PATENT APPLICATION

(11) **EP 3 597 102 A1**
(43) Date of publication of application: **22.01.2020**
(21) Application number: 18184590.0
(22) Date of filing: 19.07.2018
(51) Int. Cl.: A61B 5/04, A61B 5/0476, A61B 5/00

(54) **BRAIN COMPUTER INTERFACE**

(71) Applicant: Universiteit Antwerpen, 2000 Antwerpen (BE)
(72) Inventor: Verwulgen, Stijn, 2900 Schoten (BE); Lacko, Daniël, 2900 Schoten (BE); Vleugels, Jochen, 2140 Antwerpen (BE); Huysmans, Toon, 3073 KH Rotterdam (NL); Truijen Steven, 2980 Zoersel (BE)
(74) Representative: DenK iP

(57) **Abstract**

The present invention relates to an interfacing system for interfacing a plurality of elements to a surface of interest. The elements thereby are suitable for locally sensing a signal of the surface of interest or for locally actuating the surface of interest. The interfacing system comprises a plurality of element holders, each element holder being configured for holding an element for interfacing with the surface of interest and each element holder being configured for providing a pressure exerted on the element for interfacing the element with the surface of interest, the pressure being based on a fluidic action in the element holder. The interfacing system also comprises an outer shell interconnecting the plurality of element holders. At least two of the plurality of element holders further are fluidically interconnected so as to link the fluidic action in the at least two element holders thereby linking the pressure exerted by the element holders on the elements.

## Description

### Field of the invention

The present invention generally relates to systems for sensing or actuating on a surface of interest, such as for example for performing Electroencephalograms (EEG) or for application of current magnetic fields, or for neuro navigation. More particularly, the present invention relates to a system for providing an interface between sensing and/or actuating elements on a surface of interest, such as for example a brain computer interface, allowing to take into account inter-subject variation throughout a given target population.

### Background of the invention

Electroencephalogram (EEG) is a technique to measure brain activity through detection of small electric field fluctuations by sensitive electrodes, amplification and signal processing. EEG registration has a myriad of potential clinical and off-site applications comprising diagnosis of e.g. ALS, Alzheimer, polysomnography, epilepsy monitoring, enriched, augmented, supplemented or alternative communication, research e.g. cognitive processing and didactics, associations, commercial applications such as gaming and neuro-marketing, prosthesis steering and force feedback. The development of wearable, comfortable, easy to mount, hygienic, acceptable (non stigmatizing) and calibrated EEG headsets is needed to improve clinical use and to boost potential of-site applications. Stable electrode positioning with equal and stable pressure is needed to assure proper EEG signal acquisition, as also identified in Rozea C. et al., Journal of neural engineering (2011) 8(2) p025008. Moreover, for standardization and reliable signal acquisition, it is required that electrodes are placed at clearly defined and identifiable anatomical locations on the subject's head, the so called 10-20 system or related coordinates, as described by Oostenveld R. and Praamstra P., Clinical neurophysiology (2001) 112(4) p713-719.

3D anthropometry is the field of science that deals with the study of 3D models of the human body for the purpose of designing ergonomic products. Statistical shape models of the human skull are constructed at the university of Antwerp (Department of Product Development and Iminds-Vision lab) from medical information (CT and MRI scans) thereby excluding the effect of hair, as described in "Thickness of compressed hair layer : a pilot study in a manikin", Verwulgen et al. in 3DBST 2016; Proc. 7th Int. Conf. on 3D body scanning technologies ISBN 978-3-033-05981-8 (2016) 182-189. The model of about 100 corresponding 3D forms of the skull revealed no need for differentiation along gender. Moreover it was shown through physical experiments digital verification that a small number 3-10 of dedicated and easy to measure linear anthropometrical measurements suffices to predict a subject's skull at an accuracy (RMS) of at least 2 mm. These description of 3D surface in terms of 1D parameters allowed for the development of parametric CAD tools to develop new products that closely fits the human body with a focus on the head and skull. The relevance of the constructed models and techniques for BCI (brain computer interface) headset development was already shown in subsequent iterations of non-functional prototypes (mock-ups) with improved fit: stability and accuracy of electrode positions, in comparison with current commercial available headsets, as described in "Ergonomic design of an EEG headset using 3D anthropometry" Lacko et al. in Applied ergonomics : human factors in technology and society - ISSN 1872 - 9126 - 58(2017) 128-136.

### Summary of the invention

It is an object of embodiments of the present invention to provide good interfacing system, such as for example stable and/or accurate and/or standardized and/or repeatable interfacing system, for interfacing sensing and/or actuating elements to a surface of interest, such as e.g. brain computer interface system for interfacing with a skull, allowing to accurately accommodate inter-subject variation throughout a given target population.

It is an advantage of embodiments of the present invention that the interfacing systems can anticipate on good 3D anthropometric models of the human head, e.g. accurate and/or precise and/or representative/exhaustive 3D anthropometric models, and corresponding CAD tools. It is an advantage of embodiments of the present invention that solutions can be provided for problems identified by 3D anthropometry and visuations. It is an advantage of embodiments of the present invention that it also can be applied to body shapes that are different from the head. It is an advantage that such models and tools allow for accurate mapping of inter subject variations, such that the interfacing systems can be accurately adapted thereto.

It is an advantage of embodiments of the present invention that they can be used for capturing signals of a surface of interest, e.g. a living body such as a human body, and to provide such systems to feedback systems, such as for example in brain computer interfaces. The latter also can be applied for actuators. An example thereof is neuro-navigation wherein a pulsing magnetic field is provided to the brain, for example for reaching the motoric cortex which is then identified when a person is performing pulsating movements. The place of the magnetic field is adapted via feedback based on the motoric reactions. Such feedback systems typically require accurate positioning (both in geometry and pressure) of the sensors or actuators. Furthermore, the systems can be adapted to personal settings, which allow to make a standard system that is still user specific.

It is an advantage of embodiments of the present invention that the interfacing systems can adapt to new sizing systems e.g. of human heads. Moreover, the system can be tuned in function of new sizing systems, as it can be directly inoculated on 3D anthropometry.

It is an advantage of embodiments of the present invention that the interfacing systems take into account relevant information contained in 3D statistical shape models of the human skull.

It is an advantage of embodiments of the present invention that the interfacing systems take into account the full geometrical information contained in statistical shape models of the human body, e.g. human skull.

The present invention relates to an interfacing system for interfacing a plurality of elements to a surface of interest, the elements being suitable for locally sensing a signal of the surface of interest or for locally actuating the surface of interest,
the interfacing system comprising
- a plurality of element holders, each element holder being configured for holding an element for interfacing with the surface of interest and each element holder being configured for providing a pressure exerted on the element for interfacing the element with the surface of interest, the pressure being based on a fluidic action in the element holder,
- an outer shell interconnecting the plurality of element holders,
wherein at least two of the plurality of element holders further are fluidically interconnected so as to link the fluidic action in the at least two element holders thereby linking the pressure exerted by the element holders on the elements.
Where in embodiments of the present invention reference is made to fluidic, this can refer both to liquids or gasses.
According to embodiments of the present invention, a fluidical interconnection can induce the effect of a spring. The fluidical interconnection can buffer pressure changes. The system can for example be used in the design of wearable products to enhance usability and comfort to the wearer and enhance technical functionality of the product.
It is an advantage of embodiments of the present invention that the elements to be positioned can be positioned at reproducible geometrical points on a surface.
It is an advantage of embodiments of the present invention that contact is ensured between the elements and the surface of interest for proper interaction of the elements with the surface.
It is an advantage of embodiments of the present invention that the elements can be fitted to a variety of surfaces.
It is an advantage of embodiments of the present invention that stability of the interfacing system can be guaranteed.
It is an advantage of embodiments of the present invention that standard positioning of the interfacing elements can be guaranteed.
It is an advantage of embodiments of the present invention that accuracy of positioning of the interfacing elements can be guaranteed.
It is an advantage of embodiments of the present invention that the systems can improve comfort, functionality, reliability and/or wearability (e.g. mounting, wearing, unmounting).
The elements may be moveable along pre-set directions. The pre-set directions may be retrieved from 3D anthropometry.
The element holders may comprise a pressure transducing means for transferring pressure to the element based on the fluidic action.
It is an advantage of embodiments of the present invention that systems are provided facilitating mounting and un-mounting, since the contact points and the pressure thereon are displaceable based on pressure control.
It is an advantage of embodiments of the present invention that accurate element positioning is attained, even for repeated mounting and un-mounting by varying operators.
The pressure transducing means may be based on any of a piston principle or a syringe principle or a bellows principle.
The plurality of element holders may be distributed in two or more groups and wherein for each of the two or more groups the element holders within the group are fluidically interconnected.
It is an advantage of embodiments of the present invention that the different element holders can be distributed so that element holders supporting elements requiring a similar contact pressure to the surface of interest can be combined in one group while element holders supporting elements requiring another contact pressure can be combined in another group. In this way an accurate pressure to the surface of interest can be induced for all elements.
It is an advantage of embodiments of the present invention that equal pressure can be applied to a group of elements, e.g. electrodes of an EEG headset, for proper contacting of the surface and or providing a stable sensing signal quality, such as for example a stable EEG quality. It is to be noted that the system also may be used for sensing magnetic fields. It is to be noted that the system also may be used for sending signals to actuators. Embodiments of the present invention also may be used for neuro navigation.
It is an advantage of embodiments of the present invention that pressure on the interfacing elements can be controlled.
It is an advantage of embodiments of the present invention that pressure on the interfacing elements can be equalized, enhancing e.g. standardization, minimizing discomfort and maximizing effect of sensing and/or actuating elements.
It is an advantage of embodiments of the present invention that the pressure applied to individual elements can be limited to not be above a certain value so as to not induce discomfort by the wearer. It is an advantage of embodiments of the present invention that the pressure applied to individual elements can be at least above a certain value to assure proper contact with the surface of interest, e.g. the skull, for guaranteeing proper signal quality for the sensors or the actuators. In this way, attaining a good fit and stability is obtained.
The interfacing system furthermore may comprise a pumping means for introducing the fluidic action in the element holders.
The interfacing system furthermore may comprise a pressure sensor for sensing a pressure in the element holders so as to provide a pressure of the element to the surface of interest within a predetermined range.
The interfacing system may comprise a feedback system for controlling the contact of the element and the surface of interest. This may for example include external stimuli such as level of discomfort, it may include for example contact quality, it may include for example signal quality. It is an advantage of embodiments of the present invention that the degree of contact of the element and the surface of interest can be tested and can be adapted, e.g. by increasing the pressure, so as to guarantee accurate contact/conductance/actuating effect between the elements and the surface of interest. This feature could reduce set-up time in EEG headsets. The feedback system may control a geometrical characteristic, such as for example length, and/or a physical characteristic such as for example pressure.
The interfacing system may be a brain computer interface system and wherein the surface of interest is the skull.
The elements may comprise sensors for retrieving information of the surface of interest.
The elements may be any of electrodes for EEG acquisition, sensors for acquiring biofeedback, sensors for recording audio, sensors for recording sonar, sensors for recording visual cues, opto-electronics sensors and/or IMU's for tracking posture and/or movement.
Biofeedback may for example be any of the heart rate, blood saturation or temperature.
The elements may comprise actuators for providing actuation at the surface of interest.
The elements may be any of electro-magnetic actuators, vibrational elements for providing haptic feedback, electro-tactile actuators, transducers for audio transmission or electrodes for applying current.
Electro-magnetic actuators may for example be actuators for (repetitive) transcranial magnetic stimulation. Electrodes for applying a current may for example be electrodes for providing direct current transcranial stimulation.
it is an advantage of embodiments of the present invention that a combined functionality can be provided to some elements, such as for example combining certain elements for providing direct current stimulation as well as for obtaining EEG signals. It is an advantage of embodiments of the present invention that by combining functionalty of certain elements an enhanced control, safety and/or efficacy can be obtained.
The present invention relates to a method for adjusting an interfacing system as described above, the method comprising
- obtaining an interfacing system as described above,
- obtaining information regarding the contact between the elements and the surface of interest,
- adjusting the fluidic action taking into account said obtained information.

Particular and preferred aspects of the invention are set out in the accompanying independent and dependent claims. Features from the dependent claims may be combined with features of the independent claims and with features of other dependent claims as appropriate and not merely as explicitly set out in the claims.
For purposes of summarizing the invention and the advantages achieved over the prior art, certain objects and advantages of the invention have been described herein above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

The above and other aspects of the invention will be apparent from and elucidated with reference to the embodiment(s) described hereinafter.

### Brief description of the drawings

The invention will now be described further, by way of example, with reference to the accompanying drawings, in which:

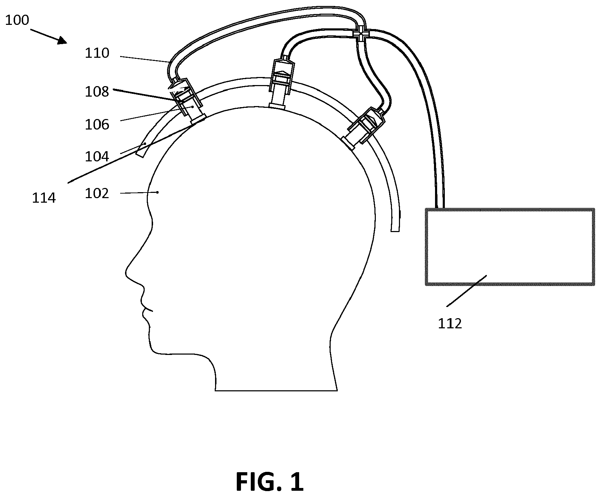

FIG. 1 shows an exemplary interfacing system according to an embodiment of the present invention.

The drawings are only schematic and are non-limiting. In the drawings, the size of some of the elements may be exaggerated and not drawn on scale for illustrative purposes. The dimensions and the relative dimensions do not necessarily correspond to actual reductions to practice of the invention.

Any reference signs in the claims shall not be construed as limiting the scope.

In the different drawings, the same reference signs refer to the same or analogous elements.

### Detailed description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.
Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.
Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.
It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.
Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments. Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.
Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.
In the description provided herein, numerous specific details are set forth. However, it is understood that embodiments of the invention may be practiced without these specific details. In other instances, well-known methods, structures and techniques have not been shown in detail in order not to obscure an understanding of this description.
In a first aspect, the present invention relates to an interfacing system for interfacing a plurality of elements to a surface of interest. The interfacing system may especially suitable for interfacing elements such as sensors or actuators with the human skull, although embodiments are not limited thereto and interfacing with other parts of a living body, e.g. human body also are envisaged. The interfacing system may be especially suitable for sensing signals, although embodiments wherein signals are sent to the living body for actuating or a combination of actuating or sensing also are envisaged. The elements thus may be suitable for locally sensing a signal of the surface of interest or for locally actuating the surface of interest. The interfacing system according to embodiments of the present invention may be adapted for sensing or actuating via electric signals, magnetic signals, electromagnetic signals, etc. The interfacing system may be used for any type of sensing and/or actuating action, such as for example Electroencephalograms (EEG) or for application of current magnetic fields, or for neuro navigation. According to embodiments of the present invention, the interfacing system comprises a plurality of element holders, each element holder being configured for holding an element for interfacing with the surface of interest. Each element holder is configured for providing a pressure exerted on the element for interfacing the element with the surface of interest. The pressure thereby is based on a fluidic action in the element holder. The interfacing system comprises an outer shell interconnecting the plurality of element holders. According to embodiments of the present invention, at least two of the plurality of element holders further are fluidically interconnected so as to link the fluidic action in the at least two element holders thereby linking the pressure exerted by the element holders on the elements.
By way of illustration, embodiments of the present invention not being limited thereto, standard and optional features of exemplary embodiments of the present invention are further discussed with reference to FIG. 1.
FIG. 1 illustrates an interface system 100 with an outer shell 104, on which element holders 106 are positioned. The outer shell 104 can be placed at a predefined location relative to a surface of interest 102. This positioning can be achieved by providing support structures that make contact with a predefined set of anatomical contact points, identifiable by geometrically characteristics of the surface. The set of anatomical contact points may for example consist of at least three nonplanar points p1, p2, p3 for unambiguous placement. The relative position can be subject/shape-dependent, e.g. in a statistical shape model defined by relative position of an individual shape for example mean shape or medoid, the other positioning defined by correspondence. The spacing can be useful to target inter-subject/shape variations and/or control positioning of elements in an individual shape. Support structures therefore may be alterable to follow the shapes along the shape model. The system therefore may be designed with an altering mechanism for making the support structures adaptable. Support structures can be endowed with additional fixation means, e.g. straps, to mount the outer shell 104 and all additional elements firmly to the surface 102.
On top of positioning and fixation of the outer shell 104 on the surface of interest 102, the outer shell 104 also acts as a housing, to contain the element holders 106. In particular, the outer shell 104 typically may contain one or more spaces in which outer regulating element holders can be mounted fixed or replaceable. Spaces may be substantially shaped as the cross section of the outer elements. Depending on the intended application, outer elements can be placed at preferred locations to target preferred point of interest.
The spaces in the outer shell 104 advantageously correspond to specific geometrical points of interest on the surface of interest 102. By mounting element holders at these points, these points can be targeted. Each point of interest has a spatial trajectory along the other shapes in the shape model. The element holders may have an axial symmetry. The axis can be oriented along the major direction of the trajectory (e.g. straight line with least square approximation, deviations from that straight line induces inaccuracy in targeting the respective point of interest). The axial length of the element holders may be related to the distance that should be covered when targeting the respective point of interest on each surface of the shape model. This distance also relates to the height of the abovementioned trajectory.
Element holders are placed in function of intended application. For example, in EEG acquisition a so-called steady visually evoked potential can be detected at the back of the skull whereby a group of element holders can be connected for proper electrode position and signal acquisition.
The element holders 106 may be adapted for being moveable along predetermined paths.
The element holder 106 may be referred to as a housing for an element, being for example a sensor or actuator. The element holder 106 comprises a pressure transducing means 108, which may be based on any of a piston principle or a syringe principle or a bellows principle. The pressure transducing means 108 are at one side interconnected with other pressure transducing means 108 through a fluidic interconnection 110, while the fluidic interconnection is at one side also connected to a pressure controller 112. The fluidic interconnection may be formed as a network of different interconnected tubes or may be a single tube linking all pressure transducing means 108. At the other side of the pressure transducing means elements such as for example a sensor and/or actuator can be positioned. The nature of the elements is directly related to the potential applications. The elements can for example be (not limitative): electrodes for EEG acquisition, vibrational elements for providing haptic feedback, sensors for acquiring biofeedback (e.g. heart rate, blood saturation, temperature), audio (microphones for audio, recording or transducers for audio transmission), sonar, visual such as for example LEDS, electro-tactile actuators opto-electronics, IMU's for posture and movement tracking, electro-magnetic actuators, .... A potential application is neuro-stimulation. Electro-magnetic actuators can be considered e.g. for (repetitive) transcranial magnetic stimulation (TMS) or direct current transcranial stimulation (tdcs), whether or not complemented with EEG measurements for enhanced control, safety, and/or efficacy. Electrodes may for example be used for applying current, e.g. for direct current transcranial stimulation. Functions of elements can be combined for enhanced control, safety and/or efficacy. The at least one element can be positioned on an organically shaped surface, being the surface of interest or a set of such corresponding surfaces constituting a statistical shape model. In further explanation, this surface of interest 102 is the human head for the purpose of clarity. This is by no means limitative for the present invention. Embodimenst of the present invention can be used to position elements on any statistical shape model of geometrically defined surfaces with one or more non-trivial radius of curvature surfaces, on controlled points of geometrical interest with controlled pressure or other controlled contact characteristics such as comfort, EEG signal quality, fixation.

The elements 114 may be positioned on the element holders 106 via a pivoting system. Any type of pivoting system may be used. In one embodiment the pivoting system may be a ball-socket joint. Such a pivoting system may ensure that elements are placed substantially perpendicular onto any shape, if desired. The range of motion is thus related to the difference in direction of the element in combination with the direction of the tangent plane at the intersection point of the axis of the element holder 106 with the surface of interest.
The pressure transducing means may in one embodiment be built up from an inner regulator and an outer regulator, fitting in each other, similar as known for example from syringes, e.g. syringes for spinal anesthesia. Both can be partially substantially shaped as cylinders. The inner regulator can move into the outer regulator to catch up any inter-shape variation. Thus space between the first end of the inner regulator and interior of outer regulator typically may vary accordingly. This space typically is sealed to prevent fluid (liquid or gas) going out. The space may also be denoted internal regulator space. At the same time, the seal must admit minimal static and dynamic friction when moving up and down.
The pressure transducing means are interconnected with fluid interconnections, allowing the pressure to be equal in the different pressure transducing means without substantial energy loss (e.g. heat dispersion as caused by friction).
The pressure in the pressure transducing means and/or fluid connections may be measured and tuned by a controller 112. The pressure controller 112 may comprise for example a processor receiving input from pressure sensors that sense the pressure in the pressure transducing means and/or fluid connections, a settable or programmed pressure level or pressure level range or profile, and it may comprise a pumping system for adding additional pressure where required and a pressure release means for releasing pressure where required.
Pressure can be limited from above not to induce discomfort by the wearer and limited from below to assure proper contact with the skull for proper signal quality, thereby attaining fit and stability. Electrode positions are attained even after repeated mounting and un-mounting by varying operators. The function of pressure controller can be multi-valent and may comprise inter-alia:
∘ displacement of inner regulators causing elements to be positioned at reproducible geometrical (anatomical) points on the surface
∘ ensuring contact for proper interaction of elements,
∘ ensuring fit on a variety of surfaces,
∘ ensuring stability,
∘ facilitating mounting and un-mounting as contact points can be displaceable by pressure control.
Pressure can be managed in function of performance of elements: signal quality, comfort, efficacy of actuator,... In some embodiments, the system is used in EEG headsets to improve comfort, functionality, reliablility and wearability: exerting equal pressure on a relevant group of electrodes for proper contact, stable EEG signal quality.
Further features in embodiments of the present invention may be as known from other interfacing systems or as described elsewhere in this description.

In a second aspect, the present invention relates to a method for adjusting an interfacing system for interfacing sensing elements or actuators with a surface of interest. The method may advantageously be applied with an interfacing system as described in the first aspect. The method comprises obtaining an interfacing system as described in the first aspect, obtaining information regarding the contact between the elements and the surface of interest, and adjusting the pressure with the controller in the fluidic connections and the pressure transducing means taking into account said obtained information. Further method steps may correspond with the functionality of the standard and optional features of the interfacing system described in the first aspect.

In a third aspect, the present invention also relates to a method for positioning sensor elements and/or actuator elements on a surface of interest. Such a method may advantageously be applied with an interfacing system as described in the first aspect. The surfaces of interest may be organic surfaces (especially surfaces having at least one non-trivial radius of curvature). One example thereof is the human body, e.g. the human head, although embodiments are not limited thereto. It is an advantage that variations can be resolved by the interfacing system according to embodiments of the present invention. Furthermore, in such systems, the element holders can be positioned at target locations for each specific surface in a reliable way. Further method steps may correspond with the functionality of the standard and optional features of the interfacing system described in the first aspect.

By way of illustration, embodiments of the present invention not being limited thereto, tests with an EEG headset according to an embodiment of the present invention is reported. In these tests, the interfacing system was used as a research instrument e.g. with pressure control and pressure measurement systems coupled to impedance/admittance/signal quality measurements, to investigate relationships between electrode pressure, comfort and functionality, in function of electrodes, position, subjects, purpose. The latter can further yield design specification and further development of wearable EEG headsets. The research system can also be applied on top of current BCI's such as medical headcaps. The influence and characteristics of various components of the interfacing system can be easily assessed and can be combined in a morphological chart as a design tool for BCI headsets.
The EEG headset actively places electrodes at standardized positions on the subject's head, where each electrode is applied with equal pressure. The interfacing system is designed for use with dry electrodes. The test results provide a better understanding on the link between general level of comfort and possible useful clear data signals, that can be used in brain computer interfaces (BCI). The test results are confined to the impact of adjustable electrodes pressure on level of user comfort only.
A challenging usability and functional factor in the deployment of dry electrodes is the pressure of the electrodes exerted on the subject's skull. On one hand, this pressure should not be too high, not to induce discomfort or annoyance by the wearer. At the other hand, a sufficiently amount of pressure is required for making stable contact with subjects' head. Also, the presence of hair is an important factor to take account of in the design of wearable EEG headsets and exerting pressure could be a solution to ensure proper contact of electrodes protruding the hair layer. Increasing electrode pressure evidently increases the chance of electrodes making proper contact with the subject's skin, thus increasing conductivity and thus increasing signal quality. So, the electrode pressure should be not too low, to ensure proper signal quality. Systems according to embodiments of the present invention allow to obtain good and accurate pressure.
Pressure requirements are, in embodiments of the present invention, integrated in other requirements for wearable EEG headsets. A particular challenge in the design of EEG head caps is placement of electrodes at pre-defined anatomical locations. These locations may be in some examples geometrically inferred from four anatomical points: nasion, inion, left pre-auriculair point and right pre-auriculair point, along the so called 10-20 system. They should be incorporated in the design of EEG headcaps to ensure accurate, standardized and repeatable EEG recording locations. In clinical applications, these positions are provided through the configuration of the system, and contact is ensured by conductive gel. In the design of wearable headsets, standardized and accurate positioning is to be guaranteed with accurate pressure range, bound from above by usability and comfort issues and bound from below by functional requirements. The latter is obtained in embodiments of the present invention.
Systems according to embodiments of the present invention also allow for investigating the pressure range since they allow for automatically positioning of the electrodes at pre-defined 10-20 locations, independent of the individual user's head size and geometry, and at the same time ensure that the same pressure is exerted, uniformly at each particular electrode location. Moreover, according to embodiments of the present invention pressure can be controlled to assess subject's discomfort and signal quality.
In the present example, geometry and sizing were based on a British adult population for male and female. Breadth of the headset was adjusted to the breadth of the head at the maximum level above and behind the ears. The maximum circumference above the brow ridges was taken as guideline for head circumference. The bitragion coronal arc measured across the crown of the head (arcwidth) provided the curvature in the frontal plane. As sagittal arc, the length of the occipital-frontal curvature from the external occipital protuberance to the glabella in the sagittal plane was taken (arclength). Size and shape of the headset were based on available classical anthropometric data of the human head and naive underlying geometrical shapes representing the human head, but the design of wearable products can be further optimized by 3D anthropometric models and methods to link univariate measurements to non-trivial geometrical shapes that accurately represents the human body shape.
Therefore, a shape model of the human skull was constructed from 100 medical images, CT and MRI scans, thereby omitting the presence of hair. The model was shown saturated for adding new skull models. Anatomical landmarks were annotated manually, e.g. inion, nasion, glabella, on which anthropometrical measurements can be inferred geometrically, e.g. head length as distance between inion and glabella. Thus, the shape model of the head was enriched with univariate measurements that allows parameterization of the human head shape. Most influencing parameters on global head shapes were retrieved: head length, bitragon width, circumference and arcwidth. Such parameters allow linking product dimensions to head shape to ensure proper fit and function.
The enriched shape model of the skull was used to design an EEG headsets at 14 electrode locations commonly used in of-the-shelve consumer headsets. The electrode variations were mapped with the shape model. This results in 10-15% improvement in fit and accuracy compared to of-the-shelf available headsets.

The test setting are using the geometry as described above. The test setting was equipped with dry spikey electrodes from the OpenBci platform (Florida Research Instruments).
A group of 12 healthy volunteers was enrolled in this study after informed consent. Before the experiments, subject completed a small survey on their age, gender, mood and experience with brain computer interfaces. Pressure is required to ensure proper connection between the electrodes and the scalp. For thicker and more voluminous the hair, more pressure is expected to ensure a good connection between scalp and the electrodes. Therefor, subjects were balanced for amount of hair. A combination of hair thickness and hair volume was assessed to that end, by making a ponytail of maximal length and measuring the circumference of the string. Three categories were distinguished: low hair volume: 5 cm and below, medium hair volume: between 5 cm and 10 cm and high hair volume: 10 cm and up. Each category contained four subjects. Tests were conducted in a space with smooth walls where the participant was sitting behind a desk in front of a laptop or handling a smartphone. In each assessment, the test device was initially placed on the subject's head without exerting any pressure. Subjects could control the pressure, ranging from 100 kPa up to 130 kPa. Pressure was bounded from below at 100 kPa by the test setting.
In the first session, the pressure was gradually increased in 12 minutes from 110 kPa to 130 kPa. In the second session, the pressure was decreased in the same time and over the same range. In both sessions, the test subject was inquired every minute on the experienced level of discomfort. At any moment, the subjects could press a panic button that elevated the pressure at once. Between the two sessions, subjects were asked to fill in a short survey on overall satisfaction with the prototype BCI headset.
In the third session, a subject specific pressure was applied for 12 minutes. The pressure was optimized for each subject, by taking the mean values of the pressures of the first quartiles discomfort. Again, subjects were inquired every minute on the experienced level of discomfort. While the test proceeded, subjects played Tetris on a smartphone, to simulate a task with high involvement. This could correspond to a real world use of wearable EEG headsets.
The pressure range where the headset was not inducing discomfort was retrieved from Likert scale levels scoring discomfort between 1 and 5. For each participant, median discomfort score was calculated. A Wilcoxon Signed Ranks Test revealed that mean of median comfort levels in sessions with increasing pressure (A) was significantly lower than in sessions with decreasing pressure (B), respectively 3.5 and 4.9. Two participants had a median of B lower than median of A, eight participants had a median of B higher than median of A and two participants had the same median for A and B.

Median discomfort level measured when the pressure is kept constant was calculated on the entire time range from 0 tot 12 minutes. A Wilcoxon Signed Rank Test showed that mean median values were significantly lower when the pressure is kept constant at subject-specific acceptable level than mean median discomfort when the pressure is increasing or decreasing, respectively 2.6, 3.5 and 4.9. Eight participants had lower medians under constant pressure than under increasing pressure, three participants had medians that scored the opposite and one participant had the same median. Two had medians at constant pressure that were higher than their medians under decreasing pressure.
Mean value of subject specific pressure was 112 kPa.
The hypothesis is that high hair volume results in lower level of discomfort. A Kruskal-Wallis Test was used on the mean level of discomfort on three different hair types. With p=0.4, the null hypothesis was not rejected. So with pressure optimized at subject-specific levels, at first quartile levels of discomfort, no influence of hair could be detected. Further research with a dedicated study design and a sufficiently amount of participants is recommended to pinpoint the effect of hair.
An ordinal regression analysis on the acquired data was performed. The null hypothesis that the variation in comfort doesn't differ significantly over time when the same pressure is applied was maintained, with 18 % of variance explained by the regression model and a 0.45 goodness of fit (Pearson). So variation in discomfort over time is not significant when constant optimized pressure is applied.
Study was limited to discomfort pain ranging from 1-10, with 1 being marked as 'comfortable', 5 'uncomfortable' and 10 as 'painful'. Most participants marked down '1' as their first reading. So they might see '1' as a baseline, to increase as the pressure went up. Others started at 5, indicating that even the first pressure level wasn't comfortable. Another limitation is that the order of test sessions was not randomized for increasing and decreasing pressure. Subjects were aware of the order in which the tests would be conducted. So there might be a co-founding factor between both test sessions.
Increasing pressure on the participants scalp is - measured by the above described pain scale - less discomfortable than decreasing pressure on the participants scalp. An evenly applied pressure of 112 kPa through the electrodes on the scalp falls within an acceptable comfort level for most subjects, during 12 minutes. Pressure for minimized discomfort could be influenced by the hair thickness of the user When the pressure stays within the acceptable range, the comfort level of the user won't change significantly over a time range of 12 minutes.

### Reference numbers

100 interface system
102 skull
104 outer shell
106 element holder
108 pressure transducing means
110 fluidic interconnection
112 presure controller
114 sensor or actuator

## Claims

1. An interfacing system for interfacing a plurality of elements to a surface of interest, the elements being suitable for locally sensing a signal of the surface of interest or for locally actuating the surface of interest,
the interfacing system comprising
- a plurality of element holders, each element holder being configured for holding an element for interfacing with the surface of interest and each element holder being configured for providing a pressure exerted on the element for interfacing the element with the surface of interest, the pressure being based on a fluidic action in the element holder,
- an outer shell interconnecting the plurality of element holders,
wherein at least two of the plurality of element holders further are fluidically interconnected so as to link the fluidic action in the at least two element holders thereby linking the pressure exerted by the element holders on the elements.

2. An interfacing system according to claim 1, wherein the elements are moveable along pre-set directions and over a pre-set range.

3. An interfacing system according to any of claims 1 or 2, wherein the element holders comprise a pressure transducing means for transferring pressure to the element based on the fluidic action.

4. An interfacing system according to any of the previous claims, wherein the pressure transducing means is based on any of a piston principle or a syringe principle or a bellows principle.

5. An interfacing system according to any of the previous claims, wherein the plurality of element holders is distributed in one, two or more groups and wherein for each of the two or more groups the element holders within the group are fluidically interconnected.

6. An interfacing system according to any of the previous claims, the interfacing system furthermore comprising a pumping means for introducing the fluidic action in the element holders.

7. An interfacing system according to any of the previous claims, the interfacing system furthermore comprising a pressure sensor for sensing a pressure in the element holders so as to provide a pressure of the element to the surface of interest within a predetermined range.

8. An interfacing system according to any of the previous claims, wherein the interfacing system comprises a feedback system for tuning the geometric and physical characteristics of elements on the surface of interest.

9. An interfacing system according to any of the previous claims, wherein the interfacing system is a brain computer interface system and wherein the surface of interest is the skull.

10. An interfacing system according to any of the previous claims, wherein the elements comprise sensors for retrieving information of the surface of interest.

11. An interfacing system according to the previous claims, wherein the elements are any of electrodes for EEG acquisition, sensors for acquiring biofeedback, sensors for recording audio, sensors for recording sonar, sensors for recording visual cues, opto-electronics sensors and/or IMU's for tracking posture and/or movement.

12. An interfacing system according to any of the previous claims, wherein the elements comprise actuators for providing actuation at the surface of interest.

13. An interfacing system according to the previous claim, wherein the elements are any of electro-magnetic actuators, vibrational elements for providing haptic feedback, electro-tactile actuators, transducers for audio transmission or electrodes for applying current.

14. An interfacing system according to any of the previous claims, wherein the system may take as an input signal a comfort level, an impedance, a signal to noise ratio, events retrieved from EEG data and neuro-navigation.

15. A method for adjusting an interfacing system according to any of claims 1 to 14, wherein the method comprises
- obtaining an interfacing system according to any of claims 1 to 14,
- obtaining information regarding the contact between the elements and the surface of interest,
- adjusting the fluidic action taking into account said obtained information.
